(19) Europäisches Patentamt
European Patent Office
Office européen des brevets

(11) Veröffentlichungsnummer : **0 005 174**
**B1**

# (12) EUROPÄISCHE PATENTSCHRIFT

(45) Veröffentlichungstag der Patentschrift : **08.07.81**

(51) Int. Cl.³ : **C 07 D285/12**// A61K31/425

(21) Anmeldenummer : **79101008.5**

(22) Anmeldetag : **03.04.79**

## (54) 2-Methyl-5-(2-hydroxystyryl)-1.3.4-thiadiazol und seine Herstellung.

(30) Priorität : **28.04.78 DE 2818766**

(43) Veröffentlichungstag der Anmeldung : **14.11.79 (Patentblatt 79/23)**

(45) Bekanntmachung des Hinweises auf die Patenterteilung : **08.07.81 Patentblatt 81/27**

(84) Benannte Vertragsstaaten : **BE CH DE FR GB NL**

(56) Entgegenhaltungen :
**DE - A - 2 132 019**
**DE - B - 1 249 873**
**HOUBEN-WEYL « Methoden der organischen Chemie »**
**4. Auflage, Band V/1b, 1972**
**GEORG THIEME VERLAG Stuttgart**
**Seiten 383 bis 385**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17 e**
**D-6710 Frankenthal (DE)**
Erfinder : **Thieme, Peter C., Dr.**
**Dr.-Hans-Hoffmann-Strasse 5**
**D-6706 Wachenheim (DE)**
Erfinder : **Franke, Albrecht, Dr.**
**Mandelring 11**
**D-6706 Wachenheim (DE)**

EP 0 005 174 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

2-Methyl-5-(2-hydroxystyryl)-1,3,4-thiadiazol und seine Herstellung

Die Erfindung betrifft das 2-Methyl-5-(2-hydroxystyryl)-1,3,4-thiadiazol der Formel 1

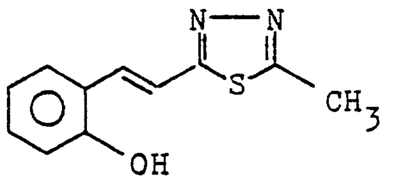

(1)

sowie Verfahren zu seiner Herstellung. Das 2-Methyl-5-(2-hydroxystyryl)-1,3,4-thiadiazol stellt ein wertvolles Zwischenprodukt für die Herstellung von pharmakologisch wirksamen Verbindungen dar.

Das 2-Methyl-5-(2-hydroxystyryl)-1,3,4-thiadiazol wird hergestellt durch Kondensation von Salicylaldehyd mit 2,5-Dimethyl-1,3,4-thiadiazol.

Die Kondensation kann durch direkte Umsetzung der beiden Ausgangsverbindungen erfolgen oder in einem inerten Lösungsmittel durchgefürt werden.

Als Lösungsmittel kommen höhersiedende Lösungsmittel, die unter Normalbedingungen eine Reaktionstemperatur von 120 bis 200 °C gestatten, in Betracht. Geeignete Lösungsmittel sind z.B. höhersiedende aromatische Kohlenwasserstoffe, wie Xylole oder Dekalin, hochsiedende Alkohole und Polyole sowie deren Äther, wie z.B. Äthylenglycol, Äthylenglykolmethyläther, sowie Mischungen der genannten Lösungsmittel. Bei der Umsetzung in einem Kohlenwasserstoff wird vorteilhafterweise das sich während der Reaktion bildende Wasser durch Auskreisen an einem Wasserabscheider entfernt.

Bei der Umsetzung werden Dimethylthiadiazol und Salicylaldehyd vorteilhafterweise in Molverhältnissen von 5 : 1 bis 2 : 1 verwendet.

Die Reaktionstemperatur für die Kondensation liegt bei Temperaturen von 120 bis 200 °C, vorteilhafterweise wird sie zwischen 140 bis 170 °C durchgeführt.

Die Kondensationsreaktion kann vorteilhafterweise mit schwachen Lewis-Säuren, wie beispielsweise Chloriden des Zinks, Zinns, Kupfers, Quecksilbers und Eisens, katalysiert werden. Besonders bevorzugt eignet sich $ZnCl_2$, wobei eine katalytische Menge von 0,1 bis 1 Mol.% zweckmäßig ist. Es sei jedoch darauf hingewiesen, daß die Reaktion mit vergleichbaren Ausbeuten auch ohne Katalysator durchgeführt werden kann.

Die Umsetzung ist im allgemeinen innerhalb von 10 bis 40 Stunden bevorzugt innerhalb von 15 bis 20 Stunden beendet. Im übrigen kann es zweckmäßig sein, die Kondensationsreaktion unter Durchleiten von einem Inertgas, z.B. Stickstoff, durchzuführen.

Es wird darauf hingewiesen, daß die erfindungsgemäße Verbindung (1) bevorzugt in der trans-Form gebildet wird. Mitgebildete cis-Form läßt sich nach an sich üblichen physikalisch-chemischen Methoden ohne Schwierigkeiten, z.B. bei der Umkristallisation, abtrennen.

Ein weiteres Verfahren zur Herstellung von 2-Methyl-5-(2-hydroxystyryl)-1,3,4-thiadiazol ist dadurch gekennzeichnet, daß man 2-Methyl-5-(triphenylphosphin-methylen)-1,3,4-thiadiazol der Formel

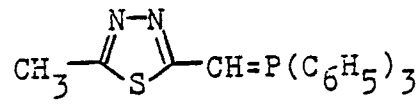

mit Salicylaldehyd umsetzt.

Diese Umsetzung erfolgt in an sich üblicher Weise, wie es beispielsweise in Houben-Weyl, Bd. 5/1 b, S. 383 ff. beschrieben wird.

Zur Lösung oder Suspension des Phosphoniumsalzes, hergestellt aus 2-Chlormethyl-5-methyl-1,3,4-thiadiazol und Triphenylphosphin in Acetonitril unter Siedetemperaturen, in alkoholischer Lösung werden nacheinander die Base zur Überführung in das Ylid und der Salicylaldehyd gegeben.

Als Alkohole kommen insbesondere Methanol und Äthanol in Betracht und als Base ist insbesondere das Alkoholat des entsprechenden Alkohols, wie Natriummethylat oder Natriumäthylat, zweckmäßig. Da die Ylide in der Regel empfindlich gegen Sauerstoff und Wasser sind, ist es zweckmäßig, in wasserfreien Lösungsmitteln und gegebenenfalls unter einem Inertgas zu arbeiten. Die Umsetzung erfolgt bei Raumtemperatur um 20 °C.

Das erfindungsgemäße 2-Methyl-5-(2-hydroxystyryl)-1,3,4-thiadiazol ist ein wertvolles Zwischenprodukt bei der Synthese pharmakologisch wirksamer Verbindungen. Beispielsweise können durch Alkylierung mit einem Epihalogenhydrin oder einem α,ω-Dihalogen-2-propanol und anschließende Umsetzung mit einem Amin Aminoderivate der allgemeinen Formel

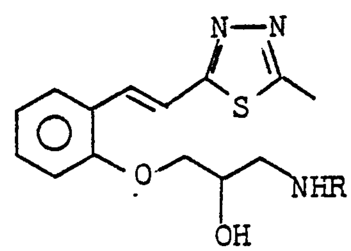

(2)

in der R insbesondere einen am zum Stickstoffatom α-ständigen Kohlenstoffatom verzweigten Alkylrest mit 3 bis 6 C-Atomen, der ggf. durch eine Alkoxygruppe mit 1 bis 3 C-Atomen substituiert ist, einen Alkenyl- oder Alkinylrest mit je 2 bis 8 C-Atomen oder Cyclopropylrest bedeutet, und deren Säureadditionssalze erhalten werden. Diese Verbindungen eignen sich aufgrund ihrer β-sympatholytischen Wirkung zur Behandlung der coronaren Herzkrankheit, von Herzrhythmusstörungen und der Hypertonie und sind Gegenstand der veröffentlichten europäischen Patentanmeldung 0 006 971.

Die folgenden Beispiele sollen das Verfahren

zur Herstellung von 2-Methyl-5-(2-hydroxystyryl)-1,3,4-thiadiazol veranschaulichen. Das 2,5-Dimethyl-1,3,4-thiadiazol wird nach literaturbekannten Methoden hergestellt, wie es in der DE-OS 21 32 019 beschrieben wird.

Beispiel 1

570 g 2,5-Dimethyl-1,3,4-thiadiazol und 275 g Salicylaldehyd werden miteinander gemischt und unter Durchleiten von Stickstoff langsam auf 150 °C erhitzt. Das Gemisch wird 30 Stunden bei 150 °C gehalten, dann abgekühlt und nach dem Abdestillieren von überschüssigem 2,5-Dimethyl-1,3,4-thiadiazol aus Methylglykol umkristallisiert. Man erhält 304 g gelbe Kristalle (56 % d. Th) vom Fp. 253 bis 254 °C.

$C_{11}H_{10}N_2OS$ (218)
ber. C 60,6 H 4,6 N 12,8 %
gef. C 59,8 H 4,6 N 12,4 %

Beispiel 2

57 g 2,5-Dimethyl-1,3,4-thiadiazol und 27,5 g Salicylaldehyd werden mit 0,5 g $ZnCl_2$ versetzt und die Mischung unter Rühren 16 Stunden bei 150 °C gehalten. Nach dem Abkühlen wird das nicht umgesetzte 2,5-Dimethyl-1,3,4-thiadiazol abdestilliert. Der Rückstand wird aus Butylacetat umkristallisiert. Es werden 35,6 g gelbe Kristalle (65 % Ausbeute) vom Schmelzpunkt 252,5 bis 254,5 °C erhalten.

Beispiel 3

57 g 2,5-Dimethyl-1,3,4-thiadiazol und 27,5 g Salicylaldehyd werden in Xylol gelöst und 48 Stunden unter Rückfluß gehalten. Während der Reaktion wird mit Hilfe eines Wasserabscheiders das gebildete Wasser ausgekreist. Nach dem Abkühlen wird das Xylol und überschüssiges Ausgangsmaterial abdestilliert. Der Rückstand wird unter Zusatz von Tierkohle aus Methylglykol umkristallisiert. Man erhält 22,3 g (41 % Ausbeute) gelbe Kristalle vom Fp. 253 bis 254 °C.

Beispiel 4

a) 2-Methyl-5-triphenylphosphoniummethyl-1,3,4-thiadiazolchlorid

148 Teile 2-Chlormethyl-5-methyl-1,3,4-thiadiazol und 261 Teile Triphenylphosphin werden in 600 Volumenteilen Acetonitril 4 Stunden unter Rückfluß erwärmt.

Nach dem Abkühlen auf 20 °C wird vom ausgefallenen Salz abfiltriert. Man erhält 348 g (85 % der Theorie) 2-Methyl-5-triphenylphosphoniummethyl-1,3,4-thiadiazolchlorid vom Fp. 288 °C (Äthanol).

b) 2-Methyl-5-(2-hydroxystyryl)-1,3,4-thiadiazol

Zu einer Suspension von 102 Teilen 2-Methyl-5-triphenylphosphoniummethyl-1,3,4-thiadiazolchlorid in 200 Volumenteilen Äthanol werden bei 20 °C 50 Teile einer 30 gewichtsprozentigen Lösung von Natriummethylat in Methanol zugegeben. Anschließend werden zu dem ausgefallenen Ylid 31 Teile Salicylaldehyd zugetropft. Nach einstündigem Rühren bei 20 °C wird der ausgefallene Feststoff abgesaugt, mit Wasser gewaschen und getrocknet. Man erhält 35 Teile (65 % der Theorie) 2-Methyl-5-(2-hydroxystyryl)-1,3,4-thiadiazol vom Fp. 269 °C (Dimethylformamid). Der höhere Schmelzpunkt zeigt, daß praktisch reine trans-Verbindung vorliegt, wie NMR-spektroskopisch nachgewiesen werden kann.

**Ansprüche**

1. 2-Methyl-5-(2-hydroxystyryl)-1,3,4-thiadiazol.

2. Verfahren zur Herstellung von 2-Methyl-5-(2-hydroxystyryl)-1,3,4-thiadiazol, *dadurch gekennzeichnet,* daß man Salicylaldehyd mit 2,5-Dimethyl-1,3,4-thiadiazol in einem Molverhältnis von 1 : 5 bis 1 : 2 oder mit 2-Methyl-5-(triphenylphosphin-methylen)-1,3,4-thiadiazol in äquimolarer Menge in an sich üblicher Weise umsetzt.

**Claims**

1. 2-Methyl-5-(2-hydroxystyryl)-1,3,4-thiadiazole.

2. A process for the preparation of 2-methyl-5-(2-hydroxystyryl)-1,3,4-thiadiazole, *characterized in that* salicylaldehyde is reacted with 2,5-dimethyl-1,3,4-thiadiazole or with 2-methyl-5-(triphenylphosphine-methylene)-1,3,4-thiadiazole in an equimolar amount in the conventional manner.

**Revendications**

1. 2-méthyl-5-(2-hydroxystyryl)-1,3,4-thiadiazole.

2. Procédé de préparation de 2-méthyl-5-(2-hydroxystyryl)-1,3,4-thiadiazole, caractérisé par le fait que l'on fait réagir, de manière, en soi, usuelle, de l'aldéhyde salycilique avec du 2,5-diméthyl-1,3,4-thiadiazole en rapport molaire de 1 : 5 à 1 : 2 ou avec du 2-méthyl-5-(triphénylphosphin-méthylen)-1,3,4-thiadiazole en quantités équimolaires.